# EUROPEAN PATENT APPLICATION

(11) **EP 2 924 124 A1**
(43) Date of publication of application: **30.09.2015**
(21) Application number: 13856389.5
(22) Date of filing: 17.10.2013
(51) Int. Cl.: C12Q 1/68

(54) **METHOD FOR THE DIAGNOSIS AND/OR PROGNOSIS OF NEURODEGENERATIVE DISEASES**

(30) Priority: 22.11.2012 ES 201231807
(71) Applicant: Consejo Superior De Investigaciones Científicas (CSIC), 28006 Madrid (ES); Centro De Investigacíon Biomédica En Red De Enfermedades Neurodegenerativas Ciberned, 28031 Madrid (ES)
(72) Inventor: TRULLAS OLIVA, Ramon, 08036 Barcelona (ES); FIGUEIRÓ SILVA, Joana, 08036 Barcelona (ES); MIHAYLOVICH PODLESNIY, Petar, 28031 Madrid (ES)
(74) Representative: Pons Ariño, Angel
(86) International application number: PCT/ES2013/070713
(87) International publication number: WO 2014/080054

(57) **Abstract**

The invention relates to the use of mitochondrial DNA as a quantitative biomarker of neurodegenerative diseases, preferably of Alzheimer disease. The invention also relates to a method and to a kit for the diagnosis and/or prognosis of said diseases using said biomarker.

LISTING OF SEQUENCES

<110> Consejo Superior de Investigaciones Científicas
      Centro de Investigación Biomédica en Red de Enfermedades
      Neurodegenerativas

<120> Method for the diagnosis and/or prognosis of neurodegenerative
diseases

<130> 1641.865

<160> 19

<170> PatentIn version 3.5

<210> 1
<211> 20
<212> DNA
<213> Artificial Sequence

<220>
<223> Forward primer F1

<400> 1
cccctgaccc ccatgcctca                                                  20

<210> 2
<211> 20
<212> DNA
<213> Artificial Sequence

<220>
<223> Reverse primer R1

<400> 2
gcggtgtggt cgggtgtgtt                                                  20

<210> 3
<211> 20
<212> DNA
<213> Artificial Sequence

<220>
<223> Forward primer F2

<400> 3
ctcactcctt ggcgcctgcc                                                  20

<210> 4
<211> 20
<212> DNA
<213> Artificial Sequence

<220>
<223>  Reverse primer R2

<400>  4
ggcggttgag gcgtctggtg                                                    20


<210>  5
<211>  14
<212>  DNA
<213>  Artificial Sequence


<220>
<223>  PROBE


<400>  5
tgccagccac cgcg                                                          14


<210>  6
<211>  20
<212>  DNA
<213>  Artificial Sequence


<220>
<223>  Forward primer 123BP F

<400>  6
ccacgggaaa cagcagtgat                                                    20


<210>  7
<211>  25
<212>  DNA
<213>  Artificial Sequence


<220>
<223>  Reverse primer 123BP R

<400>  7
ctattgactt gggttaatcg tgtga                                              25


<210>  8
<211>  36
<212>  DNA
<213>  Artificial Sequence


<220>
<223>  PROBE2


<400>  8
cgctgtagta tatccaaaga caaccatcat tccccc                                  36


<210>  9
<211>  20

<212>  DNA
<213>  Artificial Sequence

<220>
<223>  Forward primer F3

<400>  9
aaacgcctgg cagccggaag                                                   20

<210>  10
<211>  20
<212>  DNA
<213>  Artificial Sequence

<220>
<223>  Reverse primer R3

<400>  10
ggaaggggga tgcgggggaa                                                   20

<210>  11
<211>  20
<212>  DNA
<213>  Artificial Sequence

<220>
<223>  Forward primer F4

<400>  11
ctagccacac ccccacggga                                                   20

<210>  12
<211>  20
<212>  DNA
<213>  Artificial Sequence

<220>
<223>  Reverse primer R4

<400>  12
cgtatgaccg cggtggctgg                                                   20

<210>  13
<211>  20
<212>  DNA
<213>  Artificial Sequence

<220>
<223>  Reverse primer R5

<400>  13
cgggcggtgt gtgcgtactt                                                   20

<210>  14
<211>  20
<212>  DNA
<213>  Artificial Sequence

<220>
<223>  Forward primer F6

<400>  14
cgcggttcta ttttgttggt                                                    20

<210>  15
<211>  20
<212>  DNA
<213>  Artificial Sequence

<220>
<223>  Reverse primer R6

<400>  15
agtcggcatc gtttatggtc                                                    20

<210>  16
<211>  22
<212>  DNA
<213>  Artificial Sequence

<220>
<223>  Forward primer APP

<400>  16
catagcaacc gtgattgtca tc                                                 22

<210>  17
<211>  22
<212>  DNA
<213>  Artificial Sequence

<220>
<223>  Reverse primer APP

<400>  17
tggattctca tatccgttct gc                                                 22

<210>  18
<211>  24
<212>  DNA
<213>  Artificial Sequence

<220>
<223>  Forward primer IL-II

<400>  18
ctaggccaca gaattgaaag atct                                               24

<210>  19
<211>  25
<212>  DNA
<213>  Artificial Sequence

<220>
<223>  Reverse primer IL-II

<400>  19
gtaggtggaa attctagcat catcc                                          25

## Description

### NEURODEGENERATIVE DISEASES

This invention is in the field of neurodegenerative diseases, specifically in the field of methods for diagnosis and/or prognosis of these types of disease, preferably Alzheimer's disease, and is based on quantification of biomarkers in isolated biological samples.

### BACKGROUND

Recent evidence indicates that Alzheimer's disease (AD) is preceded by a long preclinical phase with abnormal biochemical, structural and functional changes in the brain. These changes suggest that the physiopathological process of neurodegeneration in AD starts long before the appearance of clinical symptoms of dementia. Therefore, identification of new biomarkers preceding the appearance of clinical symptoms of the disease is essential for making progress in understanding AD and for developing possible diagnostic and/or prognostic methods and early and efficient therapeutic treatments.

Currently two biomarkers are used in procedures to diagnose AD in a preclinical stage, before the appearance of clinical symptoms of dementia. These biomarkers are used, in the form of a diagnostic kit, by means of assays with antibodies to detect levels of amyloid-beta (Aβ) proteins and of total tau (t-tau) and phosphorylated tau (p-tau). An accumulation of Aβ in the brain or a low concentration of Aβ₁₋₄₂ in cerebrospinal fluid (CSF) or an increase in t-tau and p-tau levels in the CSF (Jack, C. R. et al., 2010, Lancet Neurol. 9, 119-128) indicate a positive diagnosis of AD. Used in combination, these two biomarkers provide a good prediction of a negative diagnosis of AD. However, the diagnosis of AD is still probabilistic and requires the presence of clinical symptoms. Furthermore, the relationship between protein levels in the brain and CSF in AD is not simple because the levels of proteins in the CSF such as total and phosphorylated tau are positively correlated whereas the levels of Aβ are negatively correlated with an accumulation in the brain and low concentration in the CSF. The reason why the accumulation of Aβ in the brain is associated with low concentrations of Aβ₁₋₄₂ in the CSF is not clear, although it may be due to a reduction in Aβ₁₋₄₂ released from the brain to the CSF (Mawuenyega, K.G. et al. 2010, Science. 330, 1774). By contrast, a high concentration of total tau in the CSF is thought to reflect a neuronal lesion associated with the accumulation of tau in the brain. However, other tau pathologies in which there is a high accumulation of tau in the neurofibrillary tangles in the brain do not show a corresponding increase in total tau in the CSF (Bian, H. et al. 2008, Neurology. 70, 1827-1835).

There is a hypothesis that mitochondrial dysfunction is involved in the physiopathology of AD, but it is unclear whether this precedes or is a consequence of the neurodegenerative process. Furthermore, AD is associated with bioenergetic and mitochondrial function abnormalities given that neurones are highly dependent on aerobic energy provided by the mitochondria (Swerdlow, R.H. et al. J. Alsheimers. Dis., 2010, 293-310).

In the state of the art it is also described that patients who suffer AD have a higher incidence of mutations in the brain in the control region of mitochondrial DNA within the elements that are known to be involved in transcription of the L-chain and replication of the H-chain (WO2007011322A2). Document WO2007011322A2 provides methods and analytical systems for pre-or post-symptomatic diagnosis of neurodegenerative disorders associated with the formation of amyloid-β deposits and/or amyloid-β fibrils through the quantification or detection of these mutations in mitochondrial DNA in the tissues or cells of an individual. Another method is described in document WO9409162A1 for diagnosis or prognosis of a predisposition to AD that comprises the detection of mutations or insertions in mitochondrial DNA.

Other studies have associated polymorphisms with AD. One hundred and thirty-eight (138) polymorphisms have been described with this disease ("Alzheimer's Disease Neuroimaging Initiative"). In their study on the role of mitochondrial haplogroups from 138 mitochondrial polymorphisms in 358 Caucasian individuals, Lakatos *et al.* provided another 5 polymorphisms to those already described that can be associated with AD (Lakatos A. et al. Neurobiol Aging, 2010, 31(8): 1355-1363). On the other hand, a study of 138 single nucleotide polymorphisms performed in 3,250 patients with AD and 1,221 controls concluded that there is no consistent evidence of association between mitochondrial DNA variations and AD.

In summary, diagnostic and/or prognostic methods have been described for AD in which the levels of biomarkers such as amyloid-β and total and phosphorylated tau are measured as well as mutations or polymorphisms in mitochondrial DNA. It is also known that the CSF is in direct contact with the brain parenchyma, so this is considered to be the best fluid for identifying possible abnormalities in brain metabolism. However, these methods have the drawback that the diagnosis and/or prognosis are performed in biological samples of tissue or CSF, which require these samples to be obtained through an invasive procedure in the patient, in addition to these methods being unreliable for early diagnosis and/or prevention of the disease. Therefore there is a need to develop other early diagnostic and/or prognostic methods for AD that can use biological samples that do not involve an invasive method for the patient. Furthermore, it would be desirable for these procedures to be simpler to perform, more sensitive, more specific, more reproducible between laboratories and with less variability than the measurement of protein levels with antibodies currently used by the kits available for the diagnosis of AD.

### DESCRIPTION OF THE INVENTION

This invention refers, in a first aspect, to the use of mitochondrial DNA as a quantitative biomarker for the diagnosis and/or prognosis of neurodegenerative diseases, preferably that of AD.

In a second aspect, this invention refers to a diagnostic and/or prognostic method *in vitro* of a neurodegenerative disease, preferably that of AD, based on the quantification of mitochondrial DNA in a biological sample isolated from a subject.

In a third aspect, this invention refers to a kit for the diagnosis and/or prognosis of a neurodegenerative disease, preferably that of AD, characterised in that it comprises at least one set of primers or probes for the quantification of mitochondrial DNA.

In a fourth aspect, this invention refers to the use of the kit comprising specific primers and/or probes for mitochondrial DNA for the diagnosis and/or prognosis of a neurodegenerative disease, preferable that of AD.

### DETAILED DESCRIPTION OF THE INVENTION

This invention proposes the use of mitochondrial DNA as a quantitative biomarker for the early diagnosis and/or prognosis of a neurodegenerative disease, preferably that of AD, and therefore provides an improved method of diagnosis and/or prognosis of a neurodegenerative disease, preferably that of AD, based on the use of this biomarker.

The method described in this invention has the advantage that it enables early diagnosis and/or prognosis of the neurodegenerative disease, that is before the appearance of the clinical symptoms associated with this disease. This would allow administering early and efficient therapeutic and/or prophylactic treatments to the patient.

Furthermore, this method can be carried out in a wide variety of biological samples without a reduction in reliability and reproducibility, including without limitation, blood, serum or plasma of the subject under study. Thus the method of the invention does not necessarily require an invasive procedure in the patient, as occurs with other diagnostic methods known to date, which need to obtain cerebral tissue or cerebrospinal fluid. Furthermore, this procedure is simple to perform, because the quantification of mitochondrial DNA can be performed through techniques widely used for nucleic acid quantification such as, for example but without limitation, PCR. The method described here is sensitive, specific, reproducible between laboratories and with less variability that the measurement of protein levels with antibodies used by currently available kits for the diagnosis of neurodegenerative diseases.

Thus, this invention is a solution to the need for providing an improved method for the early diagnosis and/or prognosis of neurodegenerative diseases, preferably that of AD, detectable in a biological sample.

Therefore, a first aspect of this invention refers to the use of mitochondrial DNA as a quantitative biomarker for the diagnosis and/or prognosis of a neurodegenerative disease, preferably that of AD.

The term "diagnosis" is considered to be the procedure through which the presence of absence of a disease is identified in a subject, preferably of a neurodegenerative disease, more preferably that of AD. The term "prognosis" refers to the procedure through which a prediction is made of the events that will occur in the development or course of a disease, preferably a neurodegenerative disease, more preferably that of AD, including but without limitation, predisposition to suffering from this disease, relapse or the ability to respond to a specific treatment.

The term "early", as used in this invention, refers to the diagnosis and/or prognosis of a disease, preferably of a neurodegenerative disease, more preferably that of AD, in a subject in the first stages of the disease, that is, before the appearance of clinical symptoms.

The term "quantitative biomarker", as used in this invention, refers to the amount or concentration of mitochondrial DNA used as an indicator of a neurodegenerative disease, preferably that of AD, and of the status of its development.

The term "mitochondrial DNA" in the present invention is the genetic material of mitochondria that can be found in both intracellularly and extracellularly or circulating. The intracellular genetic material is found in the cell and the extracellular or circulating genetic material is found outside the cell such as, but without limitation, in the cerebrospinal fluid, blood, serum, blood plasma, saliva, urine, tears or sweat. Therefore, in a preferred embodiment, mitochondrial DNA referred to in this invention is extracellular or circulating mitochondrial DNA.

In a more preferred embodiment, the regions of mitochondrial DNA that are quantified in this invention are at least one of the following: mtDNA-515, mtMNA-85, mtDNA-153 or mtDNA-123, in humans. The first three regions are located in the sequence between bases 14418 and 14932 (the exact location is shown in Table 5) of the complete Cambridge reference sequence of human mitochondrial DNA (RefSeq: NC_012920.1). These three regions code for the mitochondrial genes ND6, tRNA-Gly and CYTB; the mtDNA-123 region is between bases 805 and 927 of the complete Cambridge reference sequence of human mitochondrial DNA (RefSeq: NC_012920.1). In another preferred embodiment, the regions of mitochondrial DNA that are quantified in this invention are at least one of the following: mtDNA-115 or mtDNA-699, in mouse, that are located in the sequence that is between bases 214 and 912 (the exact location is shown in table 5) that codes mitochondrial gene for 12S ribosomal RNA according to the complete reference sequence of mouse mitochondrial DNA (RefSeq: NC_005089.1).

In this description, the term "neurodegenerative disease" is considered to be a type of disease that is classified as a type of cognitive disorder, such as for example but without limitation, Alzheimer's disease, Parkinson's disease, Creutzfeldt-Jakob disease and multiple sclerosis. These cognitive disorders are due to an increase in cell death, reducing the number of neurones and generating behavioural changes. The definition of neurodegenerative disease in this invention excludes frontotemporal dementia (FTD).

The term "Alzheimer's disease" or "AD", as used in this invention refers to a neurodegenerative disease that is manifested as a cognitive deterioration and behavioural disorder. In its typical form it is characterised by a progressive loss of memory and of other mental capacities, as the nerve cells degenerate and/or die and different areas of the brain atrophy.

The term "frontotemporal dementia" or "FTLD" for frontotemporal lobar degeneration is considered in this invention to be a clinical syndrome caused by degeneration of the frontal lobe of the human brain, that may extend to the temporal lobe. It is one of the three syndromes caused by frontotemporal lobe degeneration and the second most common cause of early dementia, after Alzheimer's disease.

In a second aspect, this invention refers to a method for the *in vitro* diagnosis and/or prognosis of a neurodegenerative disease, preferably that of AD, hereafter the "method of the invention", that comprises the following steps:
a) quantifying mitochondrial DNA in a biological sample isolated from a subject; and
b) comparing the value obtained in step (a) to a standard value.

In a preferred embodiment, the mitochondrial DNA referred to in step (a) of the method of the invention is mtDNA-515, mtDNA-85, mtDNA-123 and/or mtDNA-153 if the subject is human; mtDNA-115 and/or mtDNA-699 if the subject is mouse. In a more preferred embodiment, the mitochondrial DNA referred to in step (a) of the method of the invention is extracellular mitochondrial DNA.

The expression "to quantify" the mitochondrial DNA in a biological sample isolated from a subject, as used in this invention, refers to the measurement of the amount or the concentration. Measurement refers to the measurement of the amount or concentration of mitochondrial DNA, both intracellular and extracellular, through techniques for measuring DNA such as, for example but without limitation, PCR, quantitative real time PCR (qPCR), droplet digital PCR (ddPCR) or *Southern Blot.* Preferably, the amount quantified in this invention is expressed as the number of copies of mitochondrial DNA.

In a preferred embodiment, step (a) of the method of the invention is carried out by PCR, qPCR, ddPCR or *Southern Blot.* More preferably, step (a) is carried out through qPCR or ddPCR. Still more preferably, step (a) is carried out through ddPCR.

The term "PCR" in this invention is understood to be the polymerase chain reaction. It is a molecular biology technique where the objective is to obtain a large number of copies of a particular fragment of DNA, starting from the minimum. This technique is based on the natural property of DNA polymerases to replicate DNA strands starting from primers that are linked at the ends of the strands; this is done by using cycles of alternating high and low temperatures to separate the recently formed DNA strands after each phase of replication and joining the primer and then binding the polymerases again in order to duplicate the strands. In this invention, PCR is used for amplification and subsequent quantification of mitochondrial DNA in the biological sample, preferably for amplifying the sequences mtDNA-515, mtDNA-85, mtDNA-123 and/or mtDNA-153, if the subject is human; mtDNA-115 and/or mtDNA-699, if the subject is mouse. There are various types of PCR such as real time PCR or quantitative PCR and droplet digital PCR (ddPCR). The main characteristics of qPCR is that it allows quantifying the amount of DNA in an original sample or identifying, with very high probability, samples of specific DNA from their melting temperature. ddPCR is an improvement on the conventional polymerase chain reaction that may be used to directly quantify the nucleic acid content at a level of resolution of a single molecule and to amplify ("clonally amplify") nucleic acids such as DNA, cDNA and RNA. In this invention, the two variants of PCR can be used for quantifying mitochondrial DNA, preferably extracellular, in a biological sample.

The term *"Southern Blot",* as used in this invention refers to a molecular biology method that enables detecting the presence of a DNA sequence in a complex mixture of these nucleic acids and quantifying the DNA present in a biological sample. To do this, the technique of electrophoresis in agarose gel is used in order to separate the DNA fragments by length and, later, transfer them to a membrane in which hybridisation with a probe is performed.

The term "amount" as used in the description refers, but without limitation, to the absolute or relative amount of mitochondrial DNA, preferably expressed as the number of copies of mitochondrial DNA, as well as any other value or parameter related to this or that can be derived from this. These values or parameters comprise values of signal intensity obtained from any of the physical or chemical properties of mitochondrial DNA obtained by direct measurement, for example, values of intensity of mass spectroscopy or nuclear magnetic resonance. Additionally, these values or parameters include all those obtained through indirect measurement such as production of proteins coded for by the mtDNA, generation of free radicals or production of energy.

The term "isolated biological sample" as used in this description refers to a sample, isolated from an organism, that might come from a physiological fluid and/or any cell or tissue of the organism. In a preferred embodiment, the biological sample isolated in step (a) is selected from the list consisting of: saliva, sweat, tears, urine, cerebrospinal fluid, blood, serum and blood plasma. In a more preferred embodiment, the biological sample is cerebrospinal fluid. In another preferred embodiment, the biological sample is blood, serum or blood plasma. In a more preferred embodiment, the biological sample is serum.

The term "cerebrospinal fluid" as used in this description refers to a liquid of transparent colour that bathes the brain and spinal cord. Many diseases change its composition and its study is important and frequently determining, for the diseases of the central and peripheral nervous system. Included in these diseases are the neurodegenerative diseases such as Alzheimer's disease.

The term "standard value" is considered to mean any value or range of values derived from the quantification of mitochondrial DNA in a control biological sample from a healthy individual or in a mixture of biological samples derived from a control group.

The term "control group" in this description is understood to be a group of healthy individuals, of the same or similar age as the subject under study, from which values or ranges of values of concentration or amount of mitochondrial DNA have been obtained from the quantification of mitochondrial DNA in a collection of biological samples from these healthy individuals, and that are representative of the population in which the method of the invention is to be applied. The quantification of mitochondrial DNA must be made in the same way and be obtained from the same type of isolated biological sample as that from the subject to be studied in step (a) of the method of the invention.

The terms "healthy", "healthy individual" or "healthy subject" in this invention are considered to mean a subject or individual not suffering from a neurodegenerative disease, preferably AD.

The term "healthy population" in this invention is considered to mean a set of individuals or subjects that do not show a neurodegenerative disease, preferably AD.

The term "healthy individuals representative of the population to which the method of the invention is to be applied" is considered to mean subjects who are not suffering neurodegenerative diseases, preferably AD, at the time when a biological sample is isolated to analyse and who as a group have a pattern similar in, for example but without limitation, race, age or gender distribution, as the population of patients or subjects to which the method of the invention is to be applied.

The term "comparison" as used in this invention refers, but is not limited to, the comparison of the amount of mitochondrial DNA determined in the biological sample of step (a) with a standard value. The comparison described in step (b) of the method of the invention can be performed manually or assisted by a computer.

In another preferred embodiment, the method of the invention further comprises:
c) assigning the subject of step (a) to the group of patients with a predisposition for suffering a neurodegenerative disease, preferably that of AD, or to the group of patients suffering from a neurodegenerative disease, preferably that of AD, when the value obtained in step (a) is significantly less than the standard value.

A "significantly lower" amount than a standard value can be established by an expert in the field through the use of various statistical tools such as, for example but without limitation, by determination of confidence intervals, determination of the p value, two-tailed Student's test, Fisher's discriminant functions, using Kruskal-Wallis analysis with Dunn's post hoc multiple comparisons test, one-way ANOVA with Bonferroni's post hoc multiple comparisons test or Kruskal-Wallis U tests.

The subject referred to in step (a) of the method of the invention may be a human, but also non-human mammals such as, for example but without limitation, rodents, ruminants, felines or dogs. Therefore, in another preferred embodiment, the subject from which the biological sample of step (a) of the method of the invention is isolated is a mammal. In a more preferred embodiment, the mammal is a human.

In a third aspect, this invention refers to a kit for the diagnosis and/or prognosis of a neurodegenerative disease, preferably AD, hereafter called "kit of the invention", that comprises at least one of the following sets of primers or probes:
a) SEQ ID NO: 1 and SEQ ID NO: 4,
b) SEQ ID NO: 1 and SEQ ID NO: 2 and optionally SEQ ID NO: 8,
c) SEQ ID NO: 3 and SEQ ID NO: 4,
d) SEQ ID NO: 6 and SEQ ID NO: 7 and optionally SEQ ID NO: 5, or
e) SEQ ID NO: 9 and SEQ ID NO: 10.

In a preferred embodiment, the kit of the invention comprises the primers SEQ ID NO: 3 and SEQ ID NO: 4 or SEQ ID NO: 9 and SEQ ID NO: 10.

In a fourth aspect, this invention refers to the use of a kit comprising specific primers and/or probes for mitochondrial DNA for the diagnosis and/or prognosis of a neurodegenerative disease, preferably that of AD. In a preferred embodiment, these specific primers and/or probes for mitochondrial DNA are specific for mitochondrial DNA; mtDNA-515, mtDNA-85, mtDNA-123 and/or mtDNA-153, of human; or mtDNA-115 and/or mtDNA-699 of mouse. In a more preferred embodiment of the fourth aspect of the invention, this kit is the kit of the invention described above.

The kit of the invention may comprise, without limitation, labelled or unlabelled primers, labelled or unlabelled probes, buffers, agents for preventing contamination, marker compounds such as, for example but without limitation, fluorophores, etc. The kit of the invention may also include all the supports and recipients necessary for implementing and optimising it. The kit of the invention may contain positive and negative controls. Preferably, this kit also comprises instructions to carry out the quantification of mitochondrial DNA according to the description in this invention.

In a preferred embodiment, at least one of the sets of primers or probes of the kit of the invention is labelled or immobilised. In a more preferred embodiment, at least one of the sets of primers or probes of the kit of the invention is labelled with a label selected from the list comprising: a radioisotope, fluorescent or luminescent marker, antibody, antibody fragment, affinity label, enzyme and enzyme substrate. In a yet more preferred embodiment, at least one of the sets of primers or probes of the kit of the invention is immobilised on a support such as, for example but without limitation, in a nylon matrix, plastic support or beads.

In this description, the term "primer" is a nucleic acid chain that serves as a starting point for the amplification of DNA. It is a short nucleic acid sequence containing a free 3' hydroxyl group that forms complementary base pairs to the template strand and acts as a starting point for the addition of nucleotides in order to copy and amplify the template strand. In this invention, the primers are bound at the ends of the mitochondrial DNA for amplification and detection by, for example but without limitation, PCR.

The term "probe" as used in this description refers to a small DNA fragment labelled with fluorescence that emits signals detectable by laser and is used as a tool in, for example but without limitation, qPCR or ddPCR to quantify mitochondrial DNA in a biological sample.

Specific primers and/or probes for mitochondrial DNA, preferably for mitochondrial DNA mtADN-515, mtADN-85, mtADN-123 and/or mtADN-153, of human; mtADN-115 and/or mtADN-699 of mouse, can be designed by techniques for designing primers and/or probes that are well known in the state of the art. Preferably, the primers referred to in this invention are selected from SEQ ID NO: 1, SEQ ID NO: 2, SEQ ID NO: 3, SEQ ID NO: 4, SEQ ID NO: 6, SEQ ID NO: 7, SEQ ID NO: 9 and SEQ ID NO: 10. Preferably, the probes this invention refers to are selected from SEQ ID NO: 5 and SEQ ID NO: 8.

Throughout the description and claims, the word "comprise" and its variants does not exclude other technical characteristics, additives, components or steps. For experts in the field, other objects, advantages and characteristics of the invention will emerge, partly from the description and partly from the practice of the invention. The following examples and figures are provided for the purpose of illustration only and are not intended to limit this invention.

### BRIEF DESCRIPTION OF THE FIGURES

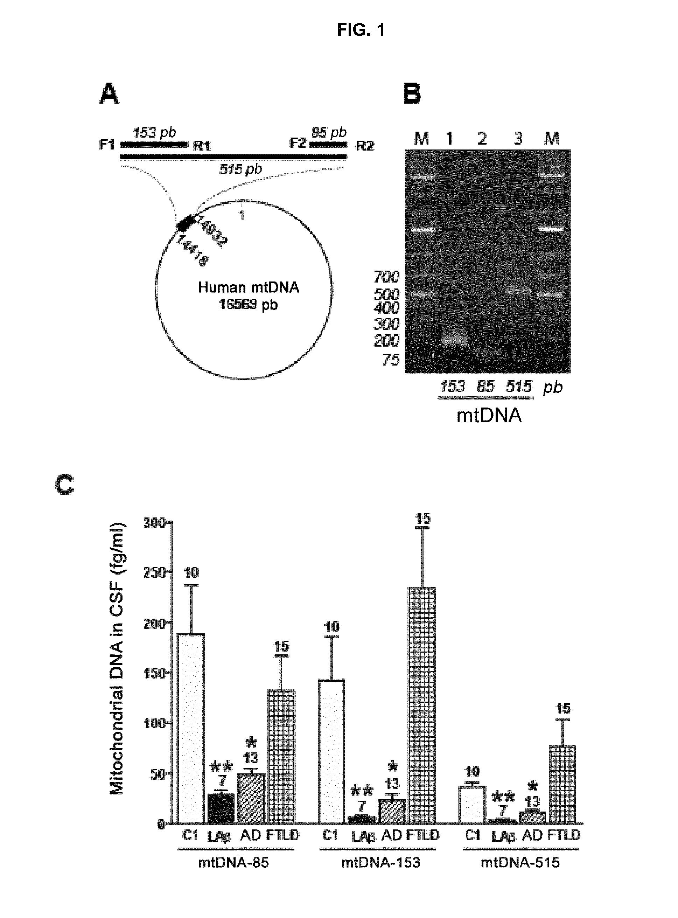

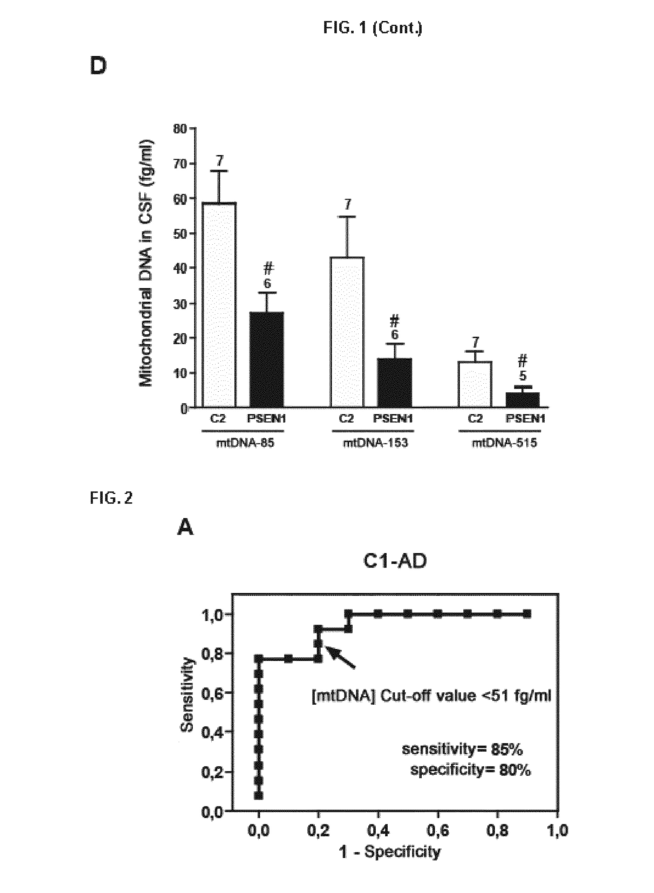

**Fig. 1****. Reduction of mitochondrial DNA concentration in the cerebrospinal fluid of subjects with preclinical Alzheimer's disease.** The concentration of mitochondrial DNA in CNF samples was measured by qPCR with primers directed to a region between bases 14418 and 14932 of the human mitochondrial DNA sequence. **A)** three different combinations of primers, two of them nested, were used to confirm the specificity of amplified mitochondrial DNA in the CSF. Two of the PCR primer pairs (SEQ ID NO: 1 / SEQ ID NO: 2 and SEQ ID NO: 3 / SEQ ID NO: 4) were designed to amplify two short sequences (153 bp and 85 bp, mitochondrial DNA-153 and mitochondrial DNA-85, respectively) located at the ends of a longer sequence, 515 bp (mitochondrial DNA-515) that in turn can be amplified by the combination of the forward and reverse primers and the second pair of primers respectively (SEQ ID NO: 1 / SEQ ID NO: 4). **B)** Representative image of an agarose gel showing the DNA size marker (M) and the mitochondrial DNA-153 (track 1), mitochondrial DNA-85 (track 2) and mitochondrial DNA-515 (track 3) products obtained respectively from three pairs of different primers in a CSF sample of subject number 2 of group C1. **C, D)** Quantification of mitochondrial DNA content by qPCR in the CSF in subjects at risk of suffering from AD (LA ), AD patients with symptoms, patients diagnosed with FTLD **(C)** and pre-symptomatic patients carrying PSEN1 mutations **(D).** The values are means ± SEM of the mitochondrial DNA concentration in the CSF expressed in fg/ml. The number above the error bars represents the number of patients per group. *, P <0.05; **, p <0.01, significantly different to the corresponding age control group (Kruskal-Wallis with Dunn's post hoc comparisons). #, P <0.05, significantly different from the corresponding age control group (two-tailed Mann-Whitney U test).

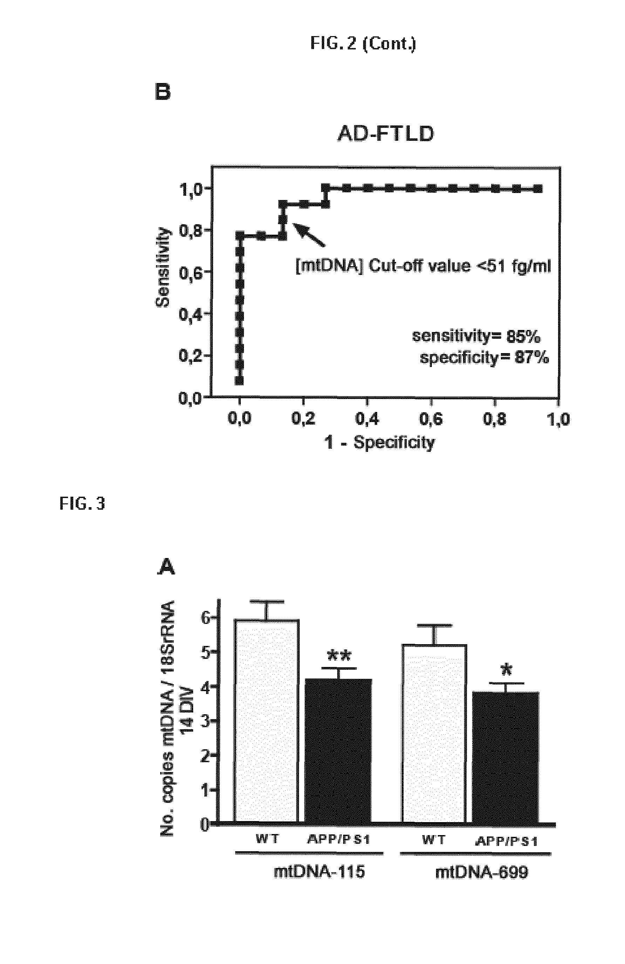

**Fig. 2****. Analysis of diagnostic discrimination by the receiver operating characteristic (ROC) curve between the AD and FTLD groups.** Sensitivity and specificity of mitochondrial DNA content in the CSF expressed in fg/ml and evaluated by qPCR with the product of the mitochondrial DNA-153 primers were determined by ROC analysis to correctly classify patients with a diagnosis of: **A)** cognitively normal controls, C1 (n=10) compared to patients diagnosed with sporadic AD, AD (n=13); the area under the curve is = 0.946, p<0.001; **B)** Patients with frontotemporal degeneration with normal AD biomarkers, FTLD (n=15) compared to patients diagnosed with sporadic AD, AD (n=13); area under the curve is = 0.959, p < 0.001.

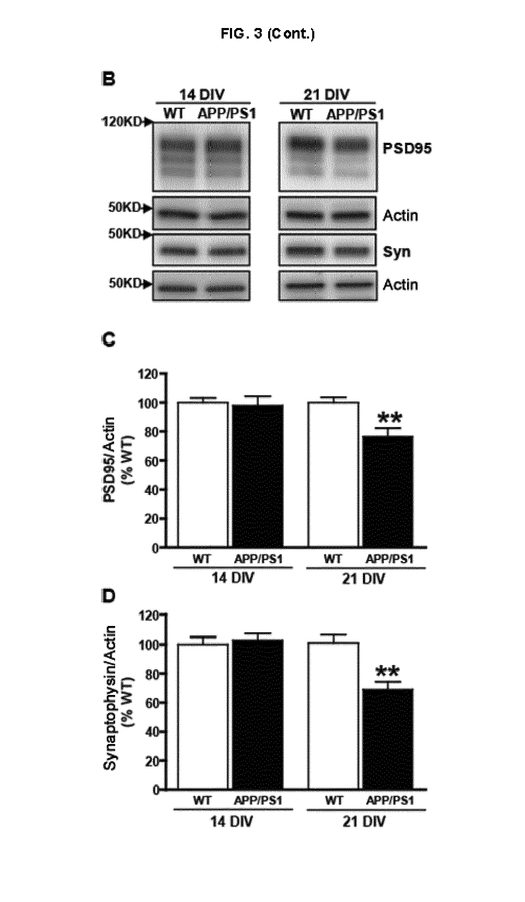

**Fig. 3****. Reduction of the number of mitochondrial DNA copies occurs before the synaptic damage in cultured cortical neurons from APP/PS1 mice.** The number of mitochondrial DNA copies and synaptic proteins were measured in cultures of wild mice cortical neurones (WT) and APP/PS1 transgenic mice. **A)** Mitochondrial DNA was measured in cultures of cortical neurones at 14 days of culture (DIV). The number of mitochondrial DNA copies and 18S rRNA were determined compared to reference plasmids containing the amplified sequences. For mitochondrial DNA, two combinations of different primers were used: mitochondrial DNA-115 and mitochondrial DNA-699, which amplify products of 115 and 699 base pairs respectively. The values are the mean ± SEM of n = number of animals, of the number of copies of mitochondrial DNA/Copies of 18S rRNA. WT (n = 8), APP/PS1 (n = 16). *, P <0.05; **, p <0.01, significantly different from the corresponding control group (two-tailed, unpaired Student's t test). **B)** Representative Western blots showing levels of PSD95 and synaptophysin (syp) proteins in lysates of cultures of WT control mice and APP/PS1 transgenic mice cortical neurones at 14 and 21 DIV. The proteins were separated by SDS-PAGE and transferred to PVDF membranes. C-D) Quantitative analysis of PDS95 and synaptophysin protein levels. The densitometric values of the bands representing immunoreactivity to PSD95(C) or synaptophysin **(D)** were normalised with values of the band of the corresponding actin immunoreactivity band. The values are mean ± SEM for PSD95 (WT n = 11 and 11; APP/PS1 DIV n = 9 and 10, 14 and 21, respectively) and for synaptophysin (WT n = 12 and 10; APP/PS1 n = 9 and 10, for 14 and 21 DIV respectively). **, P <0.01, significantly different from the corresponding control (two-tailed, unpaired Student's t test).

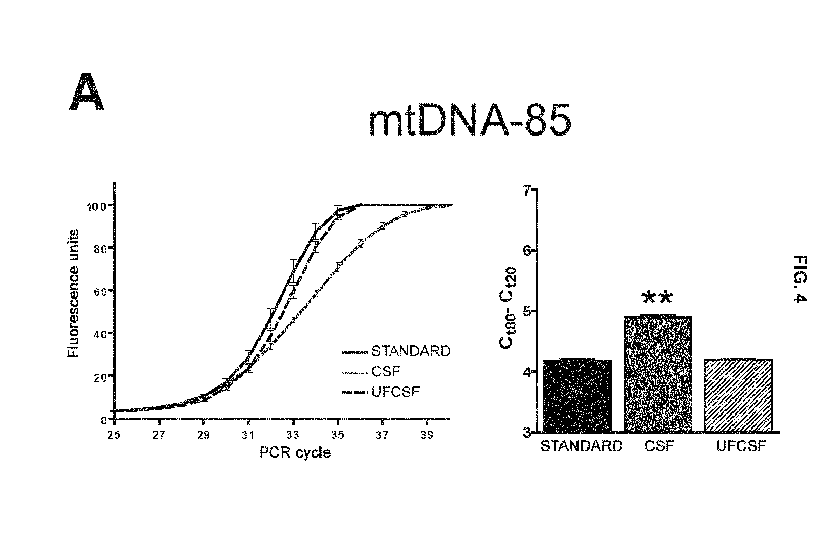

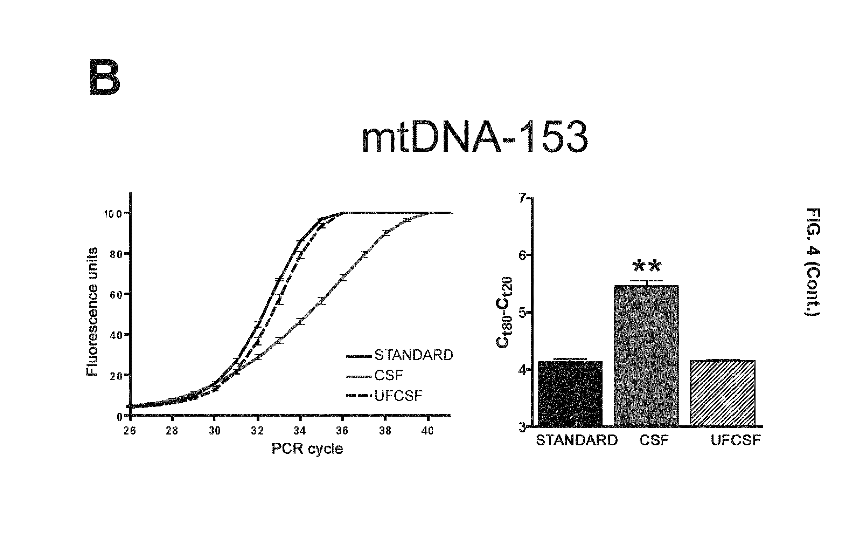

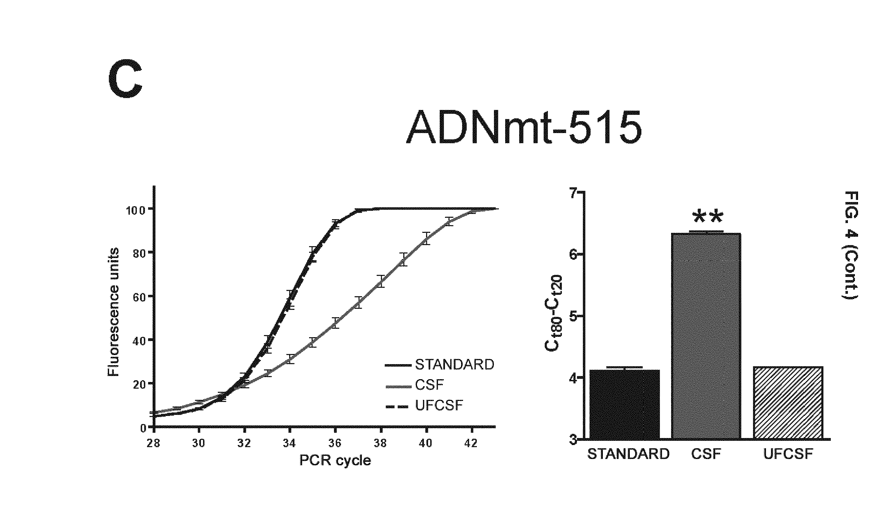

**Fig. 4****. Ultrafiltration removes the inhibitory factors of the PCR reaction from the CSF.** The qPCR amplification curves of **(A)** mitochondrial DNA-85, **(B)** mitochondrial DNA-153 and **(C)** mitochondrial DNA-515 were done, either in a calibration standard of purified human mitochondrial DNA (100 copies) of HEK293T cells (STANDARD), CSF or ultrafiltered CSF (UFCSF). Direct amplification of mitochondrial DNA in CSF gave an amplification curve with a significantly lower slope than that obtained with the human mitochondrial DNA calibration template, indicating the presence of inhibitors that reduced the efficiency of the PCR reaction and compromised precise quantification. Ultrafiltration of the CSF samples with 0.5 ml 10,000 MWCO Amicon Ultra filters completely removed the PCR amplification inhibitors, which resulted in the amplification curve slope and efficiency of PCR reaction of all the amplified products to be equivalent to that observed in the standard mitochondrial DNA. Quantitative analysis of the slopes of the PCR amplification curves, expressed in the number of cycles between 20 and 80 units of the fluorescence relative thresholds (CT80-CT20), are represented in the figures on the right of each of the mitochondrial DNA products. The values are the mean ± SEM of relative fluorescence units (left panel) or PCR cycles between threshold 20 and 80 (right panel) of n = 3 independent qPCR reactions. **, P < 0.01, significantly different from the corresponding HEK293T standard (ONEWAY ANOVA with Bonferroni post hoc comparisons).

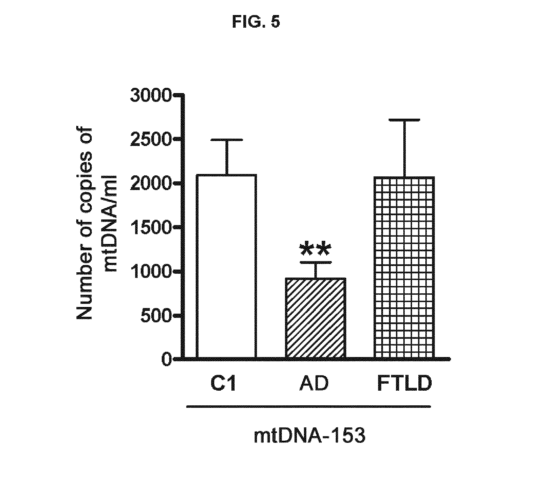

**Fig. 5****. Quantification of the number of copies of extracellular mitochondrial DNA circulating in CSF by droplet dPCR (ddPCR).** The number of copies of mitochondrial DNA were directly measured in CSF samples by ddPCR using the Bio-Rad QX100 Droplet Digital PCR platform. Amplification of mtDNA-153 was obtained with primers SEQ ID NO: 1 and SEQ ID NO: 2 and a probe labelled with FAM (SEQ ID NO: 8). The results were analysed by the QuantaSoft program. The values are expressed as mean ± SEM of the number of copies of mitochondrial DNA per ml of CSF. **C1,** control group, n = 9. **AD,** patients diagnosed with sporadic Alzheimer's disease with low levels of Aβ1-42 and high levels of t-tau and p-tau in CSF, n = 20. **FTLD,** patients diagnosed with frontotemporal degeneration and normal levels in CSF of the biomarkers related to AD, n = 11. **, P < 0.01, significantly different from C1 (Kruskal-Wallis with Dunn's post hoc comparisons).

### EXAMPLES

The invention is illustrated below with some tests performed by the inventors that demonstrate the efficacy of the use of mitochondrial DNA as a quantitative biomarker for the diagnosis and/or prognosis of neurodegenerative diseases, preferably AD. These specific examples are provided to illustrate the nature of this invention and are only included for illustrative purposes; they must not be interpreted as limiting the invention claimed here. Therefore, the examples described below illustrate the invention without limiting its field of application.

### Example 1. Study groups and techniques used in the examples.

The various subjects of the groups studied in this invention and the techniques used to carry out the examples are described in detail below.

### Subjects:

The subjects of these studies were selected from a cohort of 282 people recruited in the Alzheimer's Disease and other cognitive disorders unit from the Neurological Department of Hospital Clinic of Barcelona. All the subjects were subjected to clinical and neurophysiological evaluation and lumbar puncture. The subjects were classified by the presence or absence of dementia and by their CSF concentrations of: Aβ1-42, total amount of protein associated with tau microtubules (t-tau) and tau phosphorylated on threonine 181 (p-tau) (Tables 1 to 4). Six groups were selected for the study: 1) AD: patients diagnosed with probable AD using NINCDS/ADRDA criteria and with the typical biomarker profile that occurs in AD: low levels of Aβ1-42 and high levels of t-tau and p-tau; 2) LAß: asymptomatic subjects with risk of developing sporadic AD who do not have cognitive deficit and only low levels of Aβ1-42 in the CSF; 3) FTLD: symptomatic patients diagnosed with some of the syndromes of frontotemporal dementia including aphasia and semantic dementia and with AD biomarkers at normal levels in the CSF; 4) PSEN1: pre-symptomatic patients carrying a dominant PSEN1 mutation (M139T, K239N or I439S) with normal biomarkers in CSF and normal cognition; 5) C1: control group made up of healthy subjects without cognitive deficit and normal biomarkers in the CSF, of equivalent age to the LAß and AD groups; 6) C2: control group of healthy subjects of equivalent age to the PSEN1 mutation group without clinical, genetic or biochemical abnormalities. The pathological cut-off values used were as follows: Aβ1-42 < 500 pg/ml; t-tau > 450 pg/ml, and p-tau > 75 pg/ml. The subjects of all groups were balanced to avoid significant age differences between the groups (Tables 1 to 4). Only subjects < 75 years of age were included in this study due to the lack of healthy controls without abnormal biomarkers above this age. Finally, the samples with indicators of blood contamination or subjects with total protein above 0.7 mg/ml in the CSF were excluded from the study.

**Table 1. Age and biomarker characteristics of the study groups analysed by PCR.**

| **GROUP** | **N** | **Age** | **Aβ [pg/ml]** | **t-tau [pg/ml]** | **f-tau-T181 [pg/ml]** |
|---|---|---|---|---|---|
| | | | | | |
| C1 | 10 | 62±1 | 883±73 | 226±15 | 52±3 |
| LAβ | 7 | 67±3 | 364±32** | 162±22 | 40±6 |
| AD | 13 | 64±2 | 302±18** | 883±125** | 119±13** |
| FTLD | 15 | 61±2 | 718±40 | 268±27 | 47±3 |
| | | | | | |
| C2 | 7 | 38±3 | 690±25 | 261±17 | 53±1 |
| PSEN1 | 6 | 35±3 | 908±163 | 246±19 | 51±3 |

**Table 2. Clinical characteristics and individual values of the biomarkers of subjects analysed by qPCR.**

| **Group** | **Sample code** | **LCR Aβ [pg/ml]** | **LCR t-tau [pg/ml]** | **Alele** | **e** | **Age [Years]** | **Sex V-male M-Woman** |
|---|---|---|---|---|---|---|---|
| C1 | 1 | 860 | 289 | 62 | 3/3 | 66 | V |
| | 2 | 755 | 153 | 41 | 3/3 | 64 | M |
| | 3 | 794 | 169 | 42 | 3/3 | 62 | M |
| | 4 | 616 | 254 | 47 | 3/3 | 65 | M |
| | 5 | 750 | 197 | 44 | 3/3 | 68 | V |
| | 6 | 834 | 274 | 57 | 3/3 | 58 | M |
| | 7 | 862 | 216 | 46 | 3/3 | 66 | V |
| | 8 | 1161 | 263 | 59 | 3/3 | 57 | V |
| | 9 | 793 | 254 | 52 | 3/3 | 59 | M |
| | 10 | 1405 | 194 | 67 | 3/3 | 57 | M |
| LA_{β} | 11 | 263 | 103 | 27 | 3/3 | 55 | M |
| | 12 | 333 | 120 | 29 | 3/3 | 70 | V |
| | 13 | 274 | 127 | 26 | 3/3 | 69 | M |
| | 14 | 416 | 258 | 61 | 4/3 | 56 | M |
| | 15 | 330 | 220 | 53 | 4/3 | 73 | M |
| | 16 | 467 | 128 | 28 | 3/3 | 74 | M |
| | 17 | 466 | 179 | 55 | 3/3 | 69 | M |
| AD | 18 | 286 | 640 | 88 | 4/3 | 70 | V |
| | 19 | 338 | 2186 | 165 | 3/3 | 68 | M |
| | 20 | 206 | 899 | 127 | 4/3 | 58 | M |
| | 21 | 292 | 530 | 76 | 3/3 | 57 | M |
| | 22 | 313 | 557 | 80 | 3/3 | 72 | M |
| | 23 | 379 | 1200 | 240 | 3/3 | 60 | M |
| | 24 | 353 | 462 | 75 | 3/3 | 62 | M |
| | 25 | 317 | 987 | 146 | 4/3 | 60 | M |
| | 26 | 428 | 766 | 94 | 4/3 | 58 | V |
| | 27 | 245 | 917 | 116 | 3/3 | 69 | M |
| | 28 | 208 | 553 | 76 | 4/3 | 72 | V |
| | 29 | 243 | 754 | 126 | 4/3 | 73 | V |
| | 30 | 311 | 1024 | 144 | 3/3 | 54 | V |
| FTLD | 31 | 545 | 148 | 35 | 3/3 | 64 | M |
| | 32 | 765 | 429 | 59 | 4/3 | 54 | M |
| | 33 | 853 | 223 | 53 | 3/3 | 52 | V |
| | 34 | 922 | 192 | 44 | 3/3 | 58 | V |
| | 35 | 568 | 176 | 46 | 4/3 | 49 | V |
| | 36 | 530 | 98 | 28 | 3/3 | 65 | V |
| | 37 | 556 | 203 | 36 | 3/3 | 64 | M |
| | 38 | 785 | 281 | 53 | 4/3 | 72 | V |
| | 39 | 590 | 211 | 35 | 3/2 | 61 | M |
| | 40 | 831 | 276 | 38 | 3/3 | 58 | M |
| | 41 | 721 | 400 | 74 | 3/3 | 69 | M |
| | 42 | 652 | 430 | 58 | 3/3 | 70 | M |
| | 43 | 992 | 244 | 50 | 3/3 | 52 | V |
| | 44 | 867 | 409 | 52 | 3/3 | 52 | M |
| | 45 | 587 | 293 | 46 | 3/2 | 74 | V |
| C2 | 46 | 691 | 214 | 56 | 3/3 | 35 | M |
| | 47 | 647 | 240 | 47 | 3/3 | 35 | V |
| | 48 | 578 | 232 | 49 | 3/3 | 39 | M |
| | 49 | 668 | 348 | 57 | 3/3 | 25 | M |
| | 50 | 734 | 293 | 53 | 4/3 | 44 | M |
| | 51 | 769 | 246 | 55 | 3/3 | 42 | M |
| | 52 | 745 | 252 | 55 | 3/3 | 44 | V |
| PSEN1 | 53 | 823 | 193 | 37 | 3/3 | 24 | M |
| | 54 | 754 | 271 | 49 | 3/3 | 33 | V |
| | 55 | 656 | 324 | 54 | 3/3 | 33 | V |
| | 56 | 506 | 233 | 62 | 3/3 | 37 | V |
| | 57 | 1619 | 233 | 55 | 3/3 | 35 | M |
| | 58 | 1091 | 221 | 50 | 3/3 | 45 | M |

**Table 3. Clinical characteristics and individual values of the biomarkers in patients analysed by ddPCR.**

| **Group** | **Sample Code** | **LCR Aβ** [**pg**/**ml**] | **LCR t-tau [pg/ml]** | **LCR f-tau-T181 [pg/ml]** | **Alele ApoE** | **Age [Years]** | **Sex V-male H-female** |
|---|---|---|---|---|---|---|---|
| C1 | 1 | 860 | 289 | 62 | 3/3 | 66 | V |
| | 2 | 755 | 153 | 41 | 3/3 | 64 | F |
| | 3 | 794 | 169 | 42 | 3/3 | 62 | F |
| | 4 | 616 | 254 | 47 | 3/3 | 65 | F |
| | 5 | 750 | 197 | 44 | 3/3 | 68 | V |
| | 6 | 834 | 274 | 57 | 3/3 | 58 | F |
| | 8 | 1161 | 263 | 59 | 3/3 | 57 | V |
| | 9 | 793 | 254 | 52 | 3/3 | 59 | F |
| | 10 | 1405 | 194 | 67 | 3/3 | 57 | F |
| AD | 18 | 286 | 640 | 88 | 4/3 | 70 | V |
| | 19 | 338 | 2186 | 165 | 3/3 | 68 | F |
| | 21 | 292 | 530 | 76 | 3/3 | 57 | F |
| | 22 | 313 | 557 | 80 | 3/3 | 72 | F |
| | 23 | 379 | 1200 | 240 | 3/3 | 60 | F |
| | 24 | 353 | 462 | 75 | 3/3 | 62 | F |
| | 59 | 382 | 607 | 83 | 3/3 | 59 | F |
| | 60 | 265 | 967 | 135 | 4/3 | 64 | F |
| | 27 | 245 | 917 | 116 | 3/3 | 69 | F |
| | 25 | 317 | 987 | 146 | 4/3 | 60 | F |
| | 61 | 352 | 1638 | 221 | 3/3 | 67 | F |
| | 28 | 208 | 553 | 76 | 4/3 | 72 | V |
| | 62 | 370 | 783 | 106 | 4/3 | 69 | F |
| | 63 | 360 | 1314 | 168 | 4/3 | 67 | F |
| | 64 | 326 | 714 | 107 | 4/3 | 74 | F |
| | 29 | 243 | 754 | 126 | 4/3 | 73 | V |
| | 30 | 311 | 1024 | 144 | 3/3 | 54 | V |
| | 65 | 456 | 606 | 107 | 4/3 | 59 | V |
| | 26 | 428 | 766 | 94 | 4/3 | 58 | V |
| | 66 | 332 | 735 | 115 | 3/3 | 56 | F |
| FTLD | 31 | 545 | 148 | 35 | 3/3 | 64 | F |
| | 32 | 765 | 429 | 59 | 4/3 | 54 | F |
| | 33 | 853 | 223 | 53 | 3/3 | 52 | V |
| | 34 | 922 | 192 | 44 | 3/3 | 58 | V |
| | 35 | 568 | 176 | 46 | 4/3 | 49 | V |
| | 38 | 785 | 281 | 53 | 4/3 | 72 | V |
| | 67 | 824 | 509 | 80 | 33 | 69 | V |
| | 41 | 721 | 400 | 74 | 3/3 | 69 | F |
| | 42 | 652 | 430 | 58 | 3/3 | 70 | F |
| | 43 | 992 | 244 | 50 | 3/3 | 52 | V |
| | 44 | 867 | 409 | 52 | 3/3 | 52 | F |

**Table 4. Age and biomarker characteristics of the groups analysed by ddPCR.**

| **GRUPO** | **N** | **Edad** | **Aβ [pg/ml]** | **t-tau [pg/ml]** | **f-tau-T181 [pg/ml]** |
|---|---|---|---|---|---|
| | | | | | |
| C1 | 9 | 62±1 | 885±81 | 227±17 | 52±3 |
| AD | 20 | 65±1 | 328±14** | 897±95** | 123±10** |
| FTLD | 11 | 60±3 | 772±43 | 313±38 | 55±4 |

Subjects were included in the qPCR studies who had a sufficient amount of CSF sample to be used to measure the number of copies of mitochondrial DNA in the CSF by ddPCR. The additional subjects were included in the AD group. As in the qPCR studies, only subjects < 75 years of age were included in this study due to the lack of healthy controls above this age. **C1:** control group formed by healthy subjects without cognitive deficit, normal biomarkers in CSF and of equivalent age to the groups of patients diagnosed with AD and FTLD. **AD:** patients diagnosed with sporadic Alzheimer's disease with low levels of Aβ1-42 and high levels of t-tau and p-tau in the CSF. **FTLD:** patients diagnosed with frontotemporal lobe degeneration and with normal levels of biomarkers related to AD in the CSF. ** Significantly different from the corresponding control group (ONEWAY ANOVA with Bonferroni's post hoc comparisons, p < 0.01).

### Obtaining CSF samples:

All CSF samples were obtained with the informed consent of the subjects following the procedure approved by the Ethics Committee of Hospital Clinic of Barcelona in the Alzheimer's Disease Unit and other cognitive disorders of the Neurology Department. Samples of CSF were obtained by lumbar puncture between 09:00 and 12:00. An initial small volume of CSF was put aside to determine the presence of cells and perform other biochemical measures and then another 10 ml of CSF was collected. To avoid contamination with blood cells, possibly adhering in the lumbar puncture process, the samples of CSF were centrifuged immediately after collection at 2000 g for 10 min at 4 °C and stored at -80 °C in polypropylene tubes less than two hours after extraction. Biomarkers Aβ1-42, t-tau and p-tau were measured with immunoenzymatic assays (Innogenetics, Gante, Belgium). Amplification by PCR with primers directed to amplify a nuclear gene with a high number of copies such as 18S ribosomal RNA showed that the samples of CSF used in our experiments did not contain nuclear DNA above the detection limit of 0.6 genomes per sample, discarding the possibility of contamination of CSF with DNA of peripheral cells.

### Real time quantitative PCR:

Firstly, the circulating mitochondrial DNA concentration in CSF samples was evaluated by qPCR with a standard calibration curve. Reactions were performed in at least ten replicates in a Corbett Rotor-Gene 6000 (Corbett, Mortlake, NSW, 2137, Australia). Each 20 µl of PCR reaction consisted of 1X SsoFast EvaGreen (Bio-Rad Laboratories, Hercules, CA 94547, USA), 300 nM of each primer and 6.2 µ of the CSF sample. In order to detect mitochondrial DNA in the CSF, PCR primers were designed that amplified a region between bases 14418 and 14932, coding for mitochondrial genes ND6, tRNA-Glu and cytB, in accordance with the Cambridge reference sequence for human mitochondrial DNA (RefSeq: NC_012920.1). Primers not associated with polymorphisms of a single known nucleotide were chosen. To confirm the specificity of DNA amplification in the PCR reaction, three different combinations of primers were designed for the same region of mitochondrial DNA (Fig. 1A, B). The 5'-3' sequences of the primers are:
SEQ ID NO: 1 CCCCTGACCCCCATGCCTCA,
SEQ ID NO: 2 GCGGTGTGGTCGGGTGTGTT-,
SEQ ID NO: 3 CTCACTCCTTGGCGCCTGCC-,
SEQ ID NO: 4 GGCGGTTGAGGCGTCTGGTG-.

A combination of primers, SEQ ID NO: 1 / SEQ ID NO: 4, were designed to amplify a 515 bp long region (mitochondrial DNA-515). The other two pairs of primers, SEQ ID NO: 1 / SEQ ID NO: 2 and SEQ ID NO: 3 / SEQ ID NO: 4, were designed to amplify two short sequences (153 and 85 bp, mitochondrial DNA-153 and mitochondrial DNA-85, respectively) that are in the longer region of 515 bp (Fig. 1A). Furthermore, to verify detection of mitochondrial DNA in the CSF another PCR technique based on TaqMan probes with a combination of primers directed to a different mitochondrial gene was used. For these studies, each 20 µl of PCR reaction consisted of 1X SsoFast Supermix (Bio-Rad Laboratories, Hercules, CA 94547, USA), 100 nM of each primer, 125 nM of probe SEQ ID NO: 5 (5'-FAM-TGCCAGCCACCGCG-MGB-3') and 6.2 µl of CSF sample. The primers for this PCR reaction were designed to amplify a region between bases 805 and 927 of the mitochondrial genome coding for the mitochondrial 12S ribosomal RNA gene. The sequences of this combination of primers amplified a product of 123 bp (mitochondrial DNA-123) and are: Forward (SEQ ID NO: 6), 5'-CCACGGGAAACAGCAGTGAT-3'; Reverse (SEQ ID NO: 7), 'CTATTGACTTGGGTTAATCGTGTGA-3'.

Mitochondrial DNA can accumulate oxidative and structural damage and the longer the mitochondrial DNA sequence the higher the probability of damaged bases that might prevent amplification by polymerase during the PCR reaction. Amplification by PCR of two different short sequences with the two first combinations of primers (mitochondrial DNA-85 and mitochondrial DNA-153) was to confirm the specificity of the mitochondrial DNA target. Amplification of the longer sequence (mitochondrial DNA-515) was performed to evaluate the influence of the structural damage of mitochondrial DNA in the PCR reaction and as additional confirmation of the specificity of the target. The conditions for qPCR were optimised using the analysis of the melting temperature curve. The sizes of the amplified products and their homogeneity were estimated by gel electrophoresis. Amplification was performed using the following cycle conditions: 95 °C, 2 min followed by 45 cycles at 95 °C, 5 s, and 65 °C, 10s, 20s and 60s for mitochondrial DNA-85, mitochondrial DNA-153 and mitochondrial DNA-515 respectively. The measurements of cycle thresholds (Ct) were established in the exponential range.

Quantification of mitochondrial DNA circulating in the CSF was performed with a standard calibration curve of human mitochondrial DNA purified from HEK293T cells. Purified human mitochondrial DNA was obtained from mitochondria isolated by subcellular fractionation and Percoll gradient. Mitochondria were isolated from 1x10⁷ HEK293T cells. The cells were washed, collected in 2 ml of isolation buffer (20 mM Hepes, 300 mM sucrose, 1 mM EDTA, 1 mM DTT, pH 7.4) and were homogenised in a Potter-Elvehjem glass/teflon tissue grinder. The crude homogenate was centrifuged once at 1,000 g for 10 min to remove unlysed cells and the nuclei. The supernatant containing the crude mitochondrial fraction was placed on a discontinuous Percoll gradient, 23/15/10/3% (v/v), in isolation buffer and centrifuged at 31,000 g (Sorvall, rotor FIBERLite F21) for 5 minutes. The fraction containing mitochondria between the 15% and 23% Percoll layers was transferred to 1.5 ml microcentrifuge tubes and diluted 1:1 (v/v) in isolation buffer. After mixing gently, the mitochondria were centrifuged at 20000 g for 20 minutes. The supernatant was discarded and the DNA extracted from the mitochondrial sediment using an alkaline lysis protocol (Wizard Plus Miniprep, Promega, Madison, WI, USA). The mitochondrial DNA content in the mitochondrial DNA extract was evaluated by several methods: a) by qPCR with three combinations of primers: one combination of primers, SEQ ID NO: 1 / SEQ ID NO: 4, that amplifies the mtDNA-515 region; the combination, SEQ ID NO: 1 / SEQ ID NO: 2 that amplifies the mtDNA-153 region; and the combination, SEQ ID NO: 3 / SEQ ID NO: 4 that amplifies the mtDNA-85 region (Fig. 1A) against a standard curve of the corresponding amplified product cloned in the plasmid pJET1.2; b) by ddPCR. The concentration of mitochondrial DNA in the CSF was calculated based on the calculation that one copy of mitochondrial DNA corresponds to 18.16 [ag]. Characterisation studies indicated that mitochondrial DNA can be detected in human CSF samples between 29-35 Ct, corresponding to a range of 2-300 fg/ml. Consequently, the concentration interval of the standard calibration curve for mitochondrial DNA used in the experiments was 1-200 copies of mitochondrial DNA per reaction. The efficiency of the PCR reaction was higher than 0.95 in all samples analysed.

Table 5 below shows the primers designed and the corresponding sequences amplified.

**Table 5. Primers designed and the sequences amplified.**

| **Sequence amplified** | **Mitochondrial DNA region** | **Primer** | **SEQ ID NO** |
|---|---|---|---|
| mtDNA-153 | 14418-14570 bases of sequence NC_012920.1 | Forward primer F1 | SEQ ID NO: 1 |
| | | Reverse primer R1 | SEQ ID NO: 2 |
| mtDNA-85 | 14848-14932 bases of sequence NC_012920.1 | Forward primer F2 | SEQ ID NO: 3 |
| | | Reverse primer R2 | SEQ ID NO: 4 |
| mtDNA-515 | 14418-14932 bases of sequence NC_012920.1 | Forward primer F1 | SEQ ID NO: 1 |
| | | Reverse primer R2 | SEQ ID NO: 4 |
| mtDNA-123 | 805-927 bases of sequence NC 012920.1 | Forward primer 123BP F | SEQ ID NO: 6 |
| | | Reverse primer 123BP R | SEQ ID NO: 7 |
| mtDNA-73 | 13699-13771 bases of sequence NC_012920.1 | Direct primer F3 | SEQ ID NO: 9 |
| | | Reverse primer R3 | SEQ ID NO: 10 |
| mtDNA-115 | 214-328 bases of sequence NC_005089.1 | Direct primer F4 | SEQ ID NO: 11 |
| | | Reverse primer R4 | SEQ ID NO: 12 |
| mtDNA-699 | 214-912 bases of sequence NC_005089.1 | Direct primer F4 | SEQ ID NO: 11 |
| | | Reverse primer R5 | SEQ ID NO: 13 |
| Sequence of 219 bp of the gene coding for 18S rRNA | | Direct primer F6 | SEQ ID NO: 14 |
| | | Reverse primer R6 | SEQ ID NO: 15 |
| Transgenic APP | | Direct primer APP | SEQ ID NO: 16 |
| | | Reverse primer APP | SEQ ID NO: 17 |
| IL-II control gene | | Direct primer IL-II | SEQ ID NO: 18 |
| | | Reverse primer IL-II | SEQ ID NO: 19 |

### Digital PCR:

To validate the results obtained with real time qPCR, additional quantification of mitochondrial DNA circulating in CSF samples of some groups was performed (Tables 3 and 4) by ddPCR using the Bio-Rad QX100 Droplet Digital PCR (Bio-Rad Laboratories, Hercules, CA 94547, USA) platform. Mitochondrial DNA was directly amplified in CSF samples with a labelled FAM probe (SEQ ID NO: 8) and with primers F1 and R1, which cause amplification of the mitochondrial DNA-153 fragment used in the previous qPCR analysis. The reaction consisted of 1X ddPCR Mastermix, 900 nM of each of the primers SEQ ID NO: 1 and SEQ ID NO: 2 and 250 nM of a FAM labelled probe. Sequence SEQ ID NO: 8 of the probe was 6-carboxyfluorescein (FAM)-5'-CGCTGTAGTATATCCAAAGACAACCATCATTCCCCC-3'-BHQ-1. Drop formation was carried out by an emulsion formed by the mixture of 20 µl of the PCR reaction with 60 µl oil and the drops were generated using the QX100 microfluidic drop generator cartridge. Amplification by PCR was performed using a C1000 thermocycler with the following conditions: 95 °C 10 minutes, 40 repetitions of 94 °C 59 °C 1 min 30 sec C, 98 °C 10 min. PCR conditions were previously optimised using mitochondrial DNA purified from HEK293T cells. Presence or absence of amplification in the drops was evaluated using the QX100 Droplet Reader (FAM channel) and the results analysed using the QuantaSoft program. The results are expressed in copies of mitochondrial DNA per ml of CSF (Figure 5).

Table 6 below shows the designed probes.

**Table 6. Designed probes.**

| **Name** | **Sequence** | **SEQ ID NO** |
|---|---|---|
| PROBE | FAM-TGCCAGCCACCGCG-MGB | SEQ ID NO: 5 |
| | 888-901 bases of sequence N_012920.1_ | |
| PROBE2 | (FAM)-5'- CGCTGTAGTATATCCAAAGACAACCATCATTCCCCC- 3'-BHQ-1 | SEQ ID NO: 8 |
| | 14458-14493 bases of sequence NC_012920.1_ | |

### Ultrafiltration of CSF:

In the initial characterisation studies we observed that direct amplification of mitochondrial DNA in CSF samples produced PCR reactions with low efficiency (Fig. 4), which indicates that the CSF contains molecules that inhibit detection of DNA by PCR, compromising accurate quantification, as previously described for other qPCR assays in liquids of the human body. To achieve accurate quantification of mitochondrial DNA, we treated the CSF with various procedures to remove the inhibitors and obtain PCR efficiencies equivalent to those obtained in our standard purified mitochondrial DNA. From various procedures, we discovered that ultrafiltration of CSF completely removed inhibiting substances from the CSF and enabled a PCR reaction with an efficiency equivalent to that observed with the mtDNA standard (Fig. 4). This indicated that the inhibitory substances present in the CSF were soluble and of low molecular weight. Samples of CSF were ultrafiltered using Amicon Ultra 0.5 mL filters (Millipore, MA, USA) with 10,000 MWCO. The samples were diluted to 10 times the initial volume with water and concentrated again to the initial volume by centrifugation at 14,000 g for 10 min. This ultrafiltration process was performed twice.

### Mice genotyping:

Genomic DNA for genotyping was obtained from the tail following the Jackson Laboratory protocol (Bar Harbor, Maine, USA). A length of 1 mm of the tail was cut and incubated at 55 °C in a buffer containing 50 nM KCI, 10 mM Tris-HCl pH 8.3, 2.5 mM MgCl2•6H2O, 0.45% (v/v) of Nonidet P40, 0.45% (v/v) Tween 20 and 150 ug/ml of proteinase K (Gibco). After digestion of the tissue, the samples were incubated for 5 min at 95 °C to inactivate the proteinase K and 2 µl of this sample were used for making the genotyping for analysis by PCR. The animals were genotyped by multiplex PCR using the SsoAdvanced supermix reaction (Bio-Rad Laboratories) with primers directed to the APP transgene and to the IL-II gene as the positive control. The primers were APP: forward (SEQ ID NO: 16), 5'-CATAGCAACCGTGATTGTCATC-3' and reverse (SEQ ID NO: 17), 5'-TGGATTCTCATATCCGTTCTGC-3'; IL-II: forward (SEQ ID NO: 18), 5'-CTAGGCCACAGAATTGAAAGATCT-3' and reverse (SEQ ID NO: 19) 5'-3' GTAGGTGGAAATTCTAGCATCATCC. The PCR conditions were: 2 min at 98 °C of initial denaturation, followed by 45 cycles of 5 s of denaturation at 98 °C, 30 s of hybridisation of the primer and extension at 60 °C. The genotype was determined by analysis of the denaturation curves of the PCR products.

### Culture of cortical neurones of transgenic mice:

Primary cultures of cortical neurones were prepared from cerebral slices of E17 embryos of B6.Cg-Tg(APPswe, PSEN1dE9)85Dbo/Mmjax (APP/PS1) mice (Jackson Laboratories, Bar Harbor, ME, USA) of both sexes. The cells were dissociated in the presence of trypsin and DNase I and seeded in wells covered with poly-D-Lysine (100 µg/ml) at a density of 2 x 10^5 cells/cm^2 in neurobasal medium supplemented with 2% B27, 0.1 mg/ml gentamicin and 0.5 mM GlutaMax. A third of the volume of the medium was changed every 3-4 days. The cells were maintained at 37 °C in a humidified incubator with 5% CO2 and 95% air. The cultures were left to rest until the day of the experiments and this period of days is referred to in this invention as the days of *in vitro* (DIV) culture. All animal procedures and care was approved by the ethics committee of the University of Barcelona and were carried out in accordance with directives that conform to national (Catalonia Regional Government) and international (Guide for the care and use of laboratory animals, National Academy Press, Washington, DC, 1996) laws (Figure 4).

### Determination of the number of copies of mitochondrial DNA in cultured cortical neurones:

Total DNA was extracted by incubating cultured cortical neurones with SDS buffer (100 mM Tris-HCl, 10 mM EDTA, 0.5% SDS, 20 µg/ml RNase A, pH 8.0) for 1 hour at 37 °C in an extraction volume of approximately 1 µl per 1000 cells. The extracts were incubated with proteinase K (100 µg/ml) for 1 hour at 56 °C, followed by 10 min at 95 °C to inactivate the enzyme. The qPCR reaction was performed directly in the extraction buffer to minimise loss of mitochondrial DNA in the processes of column purification. Characterisation studies demonstrated that the extraction buffer diluted to 1:500 did not inhibit the efficiency of the PCR reaction. For amplification of mitochondrial DNA, two combinations of primers were used that amplify nested products between bases 214 to 912 of the gene coding for 12S ribosomal RNA of the complete sequence of the mitochondrial genome of *Mus musculus* with reference NC_005089.1: mitochondrial DNA-115 and mitochondrial DNA-699, that produced fragments of 115 and 699 base pairs respectively. Each reaction contained 10 µl of 2X SsoAdvanced Supermix (Bio-Rad Laboratories, Hercules, CA 94547, USA), 0.3 mM of each primer and 10 µl of a 1:250 dilution of the sample extracted in a final volume of 20 µl. The sequences of the primers are: mitochondrial DNA-115 forward (SEQ ID NO: 11) 5'-CTAGCCACACCCCCACGGGA-3' and reverse (SEQ ID NO: 12) 5'-CGTATGACCGCGGTGGCTGG-3'; mitochondrial DNA-699 the forward primer of mitochondrial DNA-115 (SEQ ID NO: 11) and the reverse (SEQ ID NO: 13) 5'-3-CGGGCGGTGTGTGCGTACTT'. The PCR conditions were: 2 min at 95 °C initial denaturation, followed by 45 cycles of 5 s of denaturation at 95 °C and 12 or 60 s of hydration of the primer and extension at 60 °C or 65 °C for mitochondrial DNA-115 and mitochondrial DNA-699 respectively. For the quantification of nuclear DNA (nDNA), we used a sequence of 219 bp of the gene coding for the 18S ribosomal RNA of which there are multiple copies in the genome. The sequences of the primers are: forward (SEQ ID NO: 14), 5'-CGCGGTTCTATTTTGTTGGT 3' and reverse (SEQ ID NO: 15) 5'-AGTCGGCATCGTTTATGGTC-3'. The PCR conditions were: 2 min at 95 °C of initial denaturation, followed by 45 cycles of 5 s of denaturation at 95 °C and 20 s of hybridisation of the primer and extension at 57 °C. All the reactions were performed in triplicate. The measurements of cycle thresholds (Ct) were established in the exponential range. Only reactions with efficiencies greater than 90% were considered. The absence of non-specific products was confirmed by analysis of denaturation curves and by gel electrophoresis. Quantification of the copies of mitochondrial DNA and 18S ribosomal RNA of nDNA was obtained by a standard curve of the respective amplification products previously cloned in pJET1.2 plasmids. The results were expressed as number of copies of mitochondrial DNA divided by the number of copies of 18S rRNA. In subsequent characterisation experiments, we determined the number of copies of 18S rRNA present in the genome of cultured cortical neurones in relation to a standard curve of two different fragments of genes with a single copy (IL-II and NP1) cloned in pJET1.2 plasmids. The results showed that cultured cortical neurones have approximately 50 copies of 18S ribosomal RNA, which indicated that these cells contain a range of 300-600 copies of mitochondrial DNA per cell (Figure 4).

### Statistical analysis:

The results are expressed as mean ± SEM. Statistical significance of the differences was examined using Kruskal-Wallis analysis with Dunn's post hoc multiple comparisons test, one-way ANOVA with Bonferroni's post hoc multiple comparisons test, or with two-tailed Student's t test or Kruskal-Wallis U test, depending on the characteristics of the samples. Analysis of the Receiver Operating Characteristic (ROC) curve and analysis of the area under the curve was performed by GraphPad Prism software.

### Example 2. Detection of extracellular mitochondrial DNA circulating in the CSF.

Firstly, the possibility of detection of extracellular mitochondrial DNA circulating in the CSF was examined. Characterisation studies showed that it is possible to detect extracellular mitochondrial DNA in a small volume (10 µl) of human CSF by PCR amplification. To confirm the specificity of DNA amplification, three different combinations of primers were used (Fig. 1); a first pair of primers (SEQ ID NO: 1 / SEQ ID NO: 4) that amplify a region of 515 base pairs long (mitochondrial DNA-515) and another two pairs of primers (SEQ ID NO: 3 / SEQ ID NO: 4 and SEQ ID NO: 1 / SEQ ID NO: 2 respectively) nested within this region that amplify 85 (mitochondrial DNA-85) and 153 (mitochondrial DNA-153) base pairs respectively (Figures 1A, B). To determine the concentration of extracellular mitochondrial DNA in the CSF, an analysis by qPCR was performed on the samples of CSF using a wide range of concentrations of an external standard of human mitochondrial DNA isolated from HEK 293T cells. The results showed that mitochondrial DNA can be detected in the CSF in a range of between 29-35 Ct, that corresponds to circulating mtDNA concentrations of between 2-300 fg/ml. Similar results were obtained with a different qPCR analysis technique based on TaqMan probes and using a combination of different primers directed at a region of mitochondrial DNA coding for the 12S ribosomal RNA mitochondrial gene.

### Example 3. Analysis of mitochondrial DNA levels circulating in CSF of asymptomatic individuals with risk of developing AD and in patients diagnosed with sporadic AD.

Next, the extracellular mitochondrial DNA content in CSF of patient with preclinical AD (chosen because they had a low level of Aβ in the CSF) and in symptomatic patients already diagnosed with sporadic AD were analysed (Table 1). Amplification of the region of mitochondrial DNA-85 revealed a marked reduction of circulating mitochondrial DNA content in CSF samples of both groups of patients: asymptomatic subjects with risk of suffering AD due to having low levels of Aß in the CSF (LAβ = 28 ± 4 fg/ml, n = 7) and symptomatic patients with a diagnosis of AD (AD = 48 ± 7 fg/ml, n = 13), corresponding to a reduction of 85 ± 2% and 74 ± 3% respectively, in comparison with the same age group control C1 (C1 = 188 ± 49 fg/ml, n = 10). An equivalent effect was observed when the mitochondrial DNA concentration in CSF was assayed with the other two combinations of primers (SEQ ID NO: 1 / SEQ ID NO: 2; and SEQ ID NO: 1 / SEQ ID NO: 4). Thus amplification by qPCR of the mitochondrial DNA-153 product showed a reduction in mitochondrial DNA content in the CSF of 96 ± 1% and 84 ± 4% in the groups LAβ (6 ± 1 fg/ml, n = 7) and AD (23 ± 6 fg/ml, n = 13), respectively, in comparison with the control group C1 (142 ± 44 fg/ml, n = 10). Similarly, amplification by qPCR of the mitochondrial DNA-515 product showed a reduction in the content of mitochondrial DNA in the CSF of 92 ± 3% and 70 ± 7% in the groups LAβ (3 ± 1 fg/ml, n = 7) and AD (11 ± 3 fg/ml, n = 13) respectively, in comparison with the control group C1 (37 ± 4 fg/ml, n = 10) (Fig. 1 C). The group of AD patients showed a tendency to have a higher mitochondrial DNA content in the CSF than the LAβ group in the three combinations of primers (SEQ ID NO: 1 / SEQ ID NO: 2; SEQ ID NO: 1 / SEQ ID NO: 4; and SEQ ID NO: 3 / SEQ ID NO: 4), but the difference was not statistically significant. In general, significant differences were not observed between the groups in the percentage reduction of mitochondrial DNA amplification between the products of greater length (mtDNA-515) and the shorter fragments (mitochondrial DNA-85 and mitochondrial DNA-153). Reduction in the amplification of mitochondrial DNA could indicate a lower number of copies of mitochondrial DNA or also loss of DNA integrity. In this latter case, it would be expected that amplification by PCR of the longer fragment would show a greater reduction because the long DNA fragments are more likely to accumulate damaged bases that prevent amplification by the polymerase. In this way, the observation that the three fragments of different length showed an equivalent reduction of mitochondrial DNA amplification in the LAβ and AD groups indicates that there is a decrease in the number of copies in these groups rather than a difference in mitochondrial DNA integrity. In summary, these results show that the mitochondrial DNA content circulating in the CSF reduces before the appearance of the clinical symptoms of AD, which indicates that the lower levels of mitochondrial DNA are associated with the risk of developing AD.

### Example 4. Differential analysis of the mitochondrial DNA levels in patients diagnosed with frontotemporal dementia and patients with AD.

To evaluate if the reduction in mitochondrial DNA content circulating in the CSF also occurs in other dementias that are not of the Alzheimer's type, we analysed the mitochondrial DNA concentration in the CSF of symptomatic patients diagnosed with frontotemporal dementia (FTLD) (Table 1). qPCR analysis revealed that the concentration of mitochondrial DNA in the CSF of these patients, measured by three combinations of different primers (mitochondrial DNA-85 = 132 ± 35 fg/ml, mitochondrial DNA-153 = 234 ± 160 fg/ml and mitochondrial DNA-515 = 77 ± 27 fg/ml, n = 15), was not significantly different to that observed in the controls C1, but was significantly higher than that observed in the LAβ and AD groups (Fig. 1 C), indicating that AD and FTLD do not share the reduction in the concentration of extracellular mitochondrial DNA in the CSF.

To determine if the amount of mitochondrial DNA in the CSF discriminates efficiently between patients with sporadic AD and controls or patients with FTLD, a statistical analysis of the receiver operating characteristic (ROC analysis) of the mitochondrial DNA content in the CSF medium with mitochondrial DNA-153 was performed (Fig. 2). The results showed that with a cut-off value of <51 fg/ml, the concentration of mitochondrial DNA in the CSF distinguished patients with AD from cognitively normal control subjects with sensitivity and specificity of 85% and 80% respectively, and with an area under the ROC curve of 0.946 (95% confidence interval = 0.86 to 1), significantly higher than chance (p < 0.001) (Fig. 2 A). Furthermore, ROC analysis showed that with a cut-off value < 51 fg/ml, the mitochondrial DNA content in the CSF distinguished the diagnosis of AS from the diagnosis of FTLD with a sensitivity of 85%, a specificity of 87% and an area under the ROC curve of 0.959 (95% confidence interval = 0.895 - 1), significantly higher than chance (p < 0.001) (Fig. 2 B). Similar results were obtained when the ROC analysis of the mitochondrial DNA content between the various groups was performed with the data of other mitochondrial DNA fragments or when the amplification data with the three pairs of primers were normalised and combined. These results provide additional evidence to suggest that a low level of mitochondrial DNA in the CSF is specifically related to AD.

### Example 5. Analysis of mitochondrial DNA in young patients without cognitive disorders and without tau and amyloid-β biomarkers but carriers of a pathogenic mutation in PSEN1.

To explore if the reduction in mitochondrial DNA circulating in the CSF also occurs in the first preclinical stages of AD, we studied the mitochondrial DNA content in the CSF of young presymptomatic patients carrying gene mutations that cause AD, and before reaching a stage when alterations of biomarkers related to AD appear. Thus, we quantified the mitochondrial DNA concentration circulating in the CSF of a group of young cognitively normal subjects who did not show any abnormality in Aß and tau biomarkers, but who had a pathogenic PSEN1 mutation (M139T, K239N or I439S) that causes the complete appearance of clinical symptoms of AD on average at least one decade later. Analysis of the CSF of these presymptomatic patients also revealed a marked reduction in the mitochondrial DNA concentration circulating in the CSF compared to the control group of corresponding age. The size of the reduction of mitochondrial DNA content in carriers of mutations compared to controls was similar in the three amplifications with different primer pairs: 54 ± 10% (PSEN1 = 27 ± 6 fg/ml, n = 6; C2 = 59 ± 9 fg/ml, n = 7), 68 ± 11% (PSEN1 = 14 ± 5 fg/ml, n = 6; C2 = 43 ± 12 fg/ml, n =7) and 66 ± 11% (PSEN1 = 4 ± 1 fg/ml, n = 5; C2 = 13 ± 3 fg/ml, n = 7) for mitochondrial DNA-85, mitochondrial DNA-153 and mitochondrial DNA-515 respectively (Fig. 1D). The results obtained in these PSEN1 presymptomatic patients are consistent with those found in subjects at risk of developing AD (Fig. 1C) and provide further evidence indicating that the reduction in the mitochondrial DNA concentration in the CSF already occurs in the first preclinical stages of AD. In contrast to the reduction in mitochondrial DNA content that was observed in the CSF of the groups of patients, it was found that in all the combinations of primers, the mitochondrial DNA content in the CSF was significantly higher (p < 0.01) in the control group of advanced age C1 (mitochondrial DNA-85 = 188 ± 49, mitochondrial DNA-153 = 142 ± 44 and mitochondrial DNA-515 = 37 ± 4 fg/ml, n = 10) compared to the control group of young age C2 (mitochondrial DNA-85 = 59 ± 9, mitochondrial DNA-153 = 43 ± 12 and mitochondrial DNA-515 = 13 ± 3 fg/ml, n = 10). This indicates that the mitochondrial DNA concentration circulating in the CSF increases with age, a process that is opposite to that found in preclinical subjects and those diagnosed with AD.

### Example 6. Analysis of the mitochondrial DNA levels circulating in the CSF by ddPCR in patients diagnosed with sporadic AD.

Next we proceeded to verify if the differences in the mitochondrial DNA content in the CSF found in our experiments could be validated with a new method of PCR, known as droplet digital PCR, which has recently been able to directly quantify nucleic acid content at the level of resolution of a single molecule. The measurement of the number of copies of mitochondrial DNA in the CSF by this new ddPCR technique using the primer pair (SEQ ID NO: 1 / SEQ ID NO: 2) and the probe SEQ ID NO: 8 that amplify mitochondrial DNA-153 confirmed that patients diagnosed with sporadic AD had a significantly lower number of mitochondrial DNA copies in the CSF (AD = 915 ± 192 copies/ml, n = 20) compared to the control group of the same age C1 and to the group with frontotemporal dementia FTLD (C1 = 2092 ± 399, n = 9; FTLD = 2053 ± 666, n = 11, copies/ml). This effect corresponds to a reduction of 56 ± 9% of mitochondrial DNA in patients with AD compared to the control group C1 (Fig. 5). These results are equivalent to those obtained using qPCR and confirm the previous experimental data that show that the mitochondrial DNA concentration in the CSF of patients diagnosed with AD is less than that in people diagnosed with FTLD.

### Example 7. Analysis of the number of copies of mitochondrial DNA per cell in cultured cortical neurones of APP/PS1 transgenic mice as an animal model of AD.

Previous studies have demonstrated that there is a reduction in the number of copies of mitochondrial DNA in the brain post mortem of patients with AD but in the human brain tissue it is not possible to determine when this effect starts and if it is caused by the neurodegenerative process. To evaluate if the reduction in the mitochondrial DNA concentration in the CSF associated with AD is caused in the nervous system before the appearance of neuronal damage, we measured the number of copies of mitochondrial DNA in cultured cortical neurones of transgenic mice that express the mutated APP/PS1 human genes. The neurones of these mice showed a significant reduction (∼28%) in the number of copies of mitochondrial DNA per cell, as measured with two combinations of different primers (SEQ ID NO:11 / SEQ ID NO:12; and SEQ ID NO:11 / SEQ ID NO:13) (Fig. 3 A). This reduction in the number of mitochondrial DNA copies was caused in cultures of cortical neurones in 14 DIV, much before the appearance of synaptic damage, measured by synaptophysin and PSD95 protein levels (Figures 3B-D). These results are consistent with the interpretation that the reduction in the mitochondrial DNA content circulating in the CSF of patients with AD reflects a smaller number of mitochondrial DNA copies per cell in the neurones of the nervous system and that the reduction in the mitochondrial DNA content appears early in the neurodegenerative process of AD.

## Claims

1. Use of mitochondrial DNA as a quantitative biomarker for the diagnosis and/or prognosis of Alzheimer's disease.

2. Use according to claim 1 wherein the mitochondrial DNA is extracellular.

3. A method for the *in vitro* diagnosis and/or prognosis of Alzheimer's disease comprising the following steps:
a) quantifying mitochondrial DNA in a biological sample isolated from a subject; and
b) comparing the value obtained in step (a) to a standard value.

4. The method according to claim 3 that additionally comprises:
c) assigning the subject of step (a) to the group of patients with predisposition for suffering Alzheimer's disease or to the group of patients suffering from Alzheimer's disease when the value obtained in step (a) is significantly less than the standard value.

5. The method according to any of claims 3 or 4 wherein the isolated biological sample is selected from the list consisting of: saliva, sweat, tears, urine, cerebrospinal fluid, blood, serum and blood plasma.

6. The method according to claim 5 wherein the isolated biological sample is cerebrospinal fluid.

7. The method according to claim 5 wherein the isolated biological sample is blood, serum or blood plasma.

8. The method according to claim 7 wherein the isolated biological sample is serum.

9. The method according to any of claims 3 to 8 wherein step (a) is carried out by PCR, quantitative PCR, droplet digital PCR or *Southern blot.*

10. The method according to claim 9 wherein step (a) is carried out by quantitative PCR or droplet digital PCR.

11. The method according to any of claims 9 or 10 wherein step (a) is carried out by droplet digital PCR.

12. The method according to any of claims 3 to 11 wherein the mitochondrial DNA is extracellular.

13. The method according to any of claims 3 to 12 wherein the subject is a human.

14. Kit for the diagnosis and/or prognosis of Alzheimer's disease that comprises at least one of the following sets of primers or probes:
a) SEQ ID NO: 1 and SEQ ID NO: 4,
b) SEQ ID NO: 1 and SEQ ID NO: 2 and optionally SEQ ID NO: 8,
c) SEQ ID NO: 3 and SEQ ID NO: 4,
d) SEQ ID NO: 6 and SEQ ID NO: 7 and optionally SEQ ID NO: 5, or
e) SEQ ID NO: 9 and SEQ ID NO: 10.

15. Kit according to claim 14 comprising the primers SEQ ID NO: 3 and SEQ ID NO: 4 or SEQ ID NO: 9 and SEQ ID NO: 10.

16. Use of a kit comprising specific primers and/or probes for mitochondrial DNA, for the diagnosis and/or prognosis of Alzheimer's disease.

17. Use according to claim 16 wherein the kit is the kit according to any of claims 14 or 15.
